# EUROPEAN PATENT SPECIFICATION

(11) **EP 4 415 845 B1**
(45) Date of publication and mention of the grant of the patent: **25.11.2026**
(21) Application number: 22797233.8
(22) Date of filing: 05.10.2022
(51) Int. Cl.: B01D 11/04, B01D 35/00, B01D 12/00, G01N 1/40, G01N 1/10, G01N 15/06, G01N 15/08

(54) **DETERMINATION OF PORE SIZE OF A MICROFILTER**
BESTIMMUNG DER PORENGRÖSSE EINER MIKROFILTRATIONSMEMBRAN
DETERMINATION DE LA TAILLE DE PORES D'UNE MEMBRANE DE MICROFILTRATION

(30) Priority: 11.10.2021 US 202163254468 P
(43) Date of publication of application: 21.08.2024
(73) Proprietor: Amgen Inc., Thousand Oaks, California 91320 (US)
(72) Inventor: HAMMOND, Matthew, Thousand Oaks, California 91320-1799 (US); ZHOU, Xiaozhu, Thousand Oaks, California 91320-1799 (US); EU, Mingda, Thousand Oaks, California 91320-1799 (US)
(74) Representative: Grünecker Patent- und Rechtsanwälte PartG mbB
(86) International application number: PCT/US2022/077622
(87) International publication number: WO 2023/064697

(56) References cited:
- WO-A1-2021/048633
- US-A- 5 480 554
- US-A1- 2005 229 681
- CALVO J I ET AL: "Comparison of liquid-liquid displacement porosimetry and scanning electron microscopy image analysis to characterise ultrafiltration track-etched membranes", JOURNAL OF MEMBRANE SCIENCE,, vol. 239, no. 2, 15 August 2004 (2004-08-15), pages 189 - 197, XP004516975, DOI: 10.1016/J.MEMSCI.2004.02.038

## Description

### FIELD

Embodiments herein relate to determination of pore sizes of microfilters, and methods of filtering cell culture products.

### BACKGROUND

Filters are used in numerous pharmaceutical and other industrial manufacturing applications. In the manufacture of therapeutic proteins such as monoclonal antibodies, the therapeutic proteins are harvested from the bioreactor in which they are produced. The harvest often utilizes a microfilter such as a hollow fiber membrane (HFM) or flat sheet membrane (FSM) filter that separates the product proteins from the cells that produce them.
US5480554 relates to an ultrafiltration/diafiltration (UF / DF) polymeric membrane that is capable of excluding 0.1 µm diameter, monodisperse, latex particles with a rejection coefficient greater than 0.998. The document describes that some manufactured membranes contain humectants such as glycerol as a pore supporting fluid. In the case of membranes containing a humectant such as glycerol, the membrane must be soaked in several changes of water or other solvent in order to remove as much of the foreign material as possible. In the method, the membrane to be tested is first thoroughly wetted with a wetting liquid that is capable of fully wetting the membrane; a displacing liquid, which is immiscible with the liquid used to wet the membrane, is placed in contact with the upstream side of the wetted membrane. Pressure is then applied to said displacing liquid and said pressure increased beyond the point where the displacing liquid begins to flow through the membrane; and the flow rate of liquid that passes through said membrane is measured as a function of the applied pressure. A plot of the flow rate of displacing liquid, per unit area of the membrane, through the membrane as a function of applied pressure is made and a straight line is drawn through the steep part of the resulting curve, which will intersect the horizontal axis at a given pressure value, which is then deemed the knee-UF (KUF) value. The KUF value or characteristic pressure is a read from the graph at the point where the straight line crosses the pressure axis and is a measure of the molecular weight cut-off of the membrane.

### SUMMARY

In some aspects, a method of determining a pore size profile of a microfilter is described, which is as follows:
A method of determining a pore size profile of a microfilter, the method comprising:
providing a saturated porous microfilter membrane comprising a first surface, a second surface, and a matrix disposed therebetween, wherein the matrix is saturated by a storage solution, wherein the storage solution comprises or consists of a water soluble non-volatile solution which is a polyol, preferably glycerin;
contacting the saturated porous microfilter membrane by immersing in an intermediate solvent, whereby the storage solution dissolves in the intermediate solvent, thereby de-saturating the membrane;
applying a test fluid to the de-saturated membrane, thereby re-saturating the membrane;
applying a pressure to the first surface of the re-saturated membrane, wherein the pressure is applied by contacting the first surface with a gas or liquid;
detecting a flow of the gas, liquid, and/or test fluid from the second surface in response to said pressure; and
determining the pore size profile of the porous microfilter membrane based on a level of the pressure that results in the flow of the gas, liquid, and/or test fluid from the second surface,
wherein de-saturating the microfilter membrane comprises the storage solution being below a limit of detection by ATR-FTIR spectroscopy.
The method comprises providing a saturated porous microfilter membrane comprising a first surface, a second surface, and a matrix disposed therebetween, in which the matrix is saturated by a storage solution, wherein the storage solution comprises or consists of a water soluble non-volatile solution which is a polyol, preferably glycerin. The method further comprises contacting the saturated porous microfilter membrane with an intermediate solvent, so that the storage solution dissolves in the intermediate solvent, thus de-saturating the membrane. The method further comprises applying a test fluid to the de-saturated membrane, thus re-saturating the membrane. The method further comprises applying a pressure to the first surface of the re-saturated membrane, in which the pressure is applied by contacting the first surface with a gas or liquid. The method further comprises detecting a flow of the gas, liquid, and/or test fluid from the second surface in response to said pressure. The method further comprises determining the pore size profile of the porous microfilter membrane based on a level of the pressure that results in the flow of the gas, liquid, and/or test fluid from the second surface. In the method, de-saturating the microfilter membrane comprises the storage solution being below a limit of detection by ATR-FTIR spectroscopy.

In some aspects, a method of filtering a cell culture product comprising a therapeutic protein is described. The method comprises providing a saturated porous microfilter membrane comprising a first surface, a second surface, and a matrix disposed therebetween, in which the matrix is saturated by a storage solution, wherein the storage solution comprises or consists of a water soluble non-volatile solution which is a polyol, preferably glycerin. The method further comprises contacting the saturated porous microfilter membrane with an intermediate solvent, in which the storage solution dissolves in the intermediate solvent, thus de-saturating the membrane. The method further comprises applying a test fluid to the de-saturated membrane, thus re-saturating the membrane. The method further comprises applying a pressure to the first surface of the re-saturated membrane, in which the pressure is applied by contacting the first surface with a gas or liquid. The method further comprises detecting a flow of the gas, liquid, and/or test fluid from the second surface in response to the pressure, in which the pore size profile of the membrane is determined based on a level of the pressure that results in the flow of the gas, liquid, and/or test fluid from the second surface. The method further comprises selecting the membrane for use in filtering only if the determined pore size profile of the membrane is within a specified range, in which the specified range accommodates passage of the therapeutic protein through the porous microfilter membrane. In the method, de-saturating the microfilter membrane comprises the storage solution being below a limit of detection by ATR-FTIR spectroscopy. The method further comprises filtering the cell culture product comprising the therapeutic protein through the selected microfilter membrane or a microfilter membrane of the same batch as the selected microfilter membrane. In some embodiments, the specified range accommodates passage of molecules having a molecular weight of up to 50 kDa, 75 kDa, 100 kDa, 150 kDa, 200 kDa, 500 kDa, 750kDa, or 1000 kDa through the porous microfilter membrane. For example, the specified range may be 5-120 nanometers, 60-100 nanometers, 5-100 nanometers, or 60-120 nanometers. In some methods of filtering the cell culture product, the cell culture product comprises cell debris and host cell protein in addition to the therapeutic protein. For any of the methods of filtering a cell culture product described herein, the cell culture product may be of a cell culture selected from the group consisting of: mammalian cells such as Chinese Hamster Ovary (CHO) cells, HeLa cells, baby hamster kidney (BHK) cells, monkey kidney cells (COS), human hepatocellular carcinoma cells (e.g., Hep G2), or human epithelial kidney 293 cells; insect cells, such as Sf21/Sf9, or *Trichoplusia* ni Bti-Tn5bl-4; yeast cells, such as *Saccharomyces* or *Pichia;* plant cells; chicken cells; and prokaryotic cells such as *Escherichia coli* cells. any of the methods of filtering a cell culture product described herein, the therapeutic protein is selected from the group consisting of: an antibody, an antigen-binding antibody fragment, an antibody protein product, a Bi-specific T cell engager (BiTE^{®}) molecule, a multispecific antibody, an Fc fusion protein, a recombinant protein, and an active fragment of a recombinant protein.

Any of the methods described herein may further comprise, prior to contacting the saturated microfilter membrane with the intermediate solvent, cutting the porous microfilter membrane to a specified dimension, such as a length of 5-15 cm. For example, the microfilter membrane may comprise flat sheet fibers, and the specified dimension may comprise a specified length and a specified width.

For any of the methods described herein, the microfilter membrane may be an ultrafiltration membrane or portion thereof.

For any of the methods described herein, the microfilter membrane may comprise, consist essentially of, or consist of polysulfone, polyethersulfone, polyvinylidene fluoride, or cellulose.

For any of the methods described herein, the storage solution comprises or consists of a water-soluble non-volatile solution, which is a polyol. By way of example, the polyol may comprise or consist of glycerol, such as glycerin. For any of the methods described herein, the storage solution may be soluble in the intermediate solvent, wherein the intermediate solvent does not dissolve the microfilter membrane, and wherein the intermediate solvent vaporizes at 1 atm pressure and 20° C.

For any of the methods described herein, the intermediate solvent may comprise or consist of an alcohol such as isopropyl alcohol.

For any of the methods described herein, the method may further comprise drying the de-saturated microfilter membrane prior to applying the test fluid.

For any of the methods described herein, de-saturating the microfilter membrane comprises the storage solution being below a limit of detection by attenuated total reflection-Fourier transform infrared (ATR-FTIR) spectroscopy, such as when an ATR-FTIR spectrum of the first and/or second surface matches a reference spectrum of pure material of which the microfilter membrane is made, such as polysulfone or polyethersulfone.

For any of the methods described herein, the drying may be performed until the intermediate solvent is below a limit of detection by ATR-FTIR spectroscopy.

For any of the methods described herein, the drying is performed until the amount of storage solution in the matrix is no more than 1%, 0.5%, 0.1%, or 0.01% of saturation.

For any of the methods described herein, the test fluid has a surface tension less than 70 mN m⁻¹.

For any of the methods described herein, the test fluid may comprise an organic solvent or mixture of organic solvents, and/or the test fluid comprises or consists of Porofil^{®} product, Fluorinert^{®} product, Porefil^{®} product, Porewick^{®} product, or Galwick^{®} product.

For any of the methods described herein, a contact angle of the test fluid to the first surface may be sufficient to saturate the filter with test fluid, such as an angle that is no more than 15°, such as an angle of 0°.

For any of the methods described herein, the microfilter membrane may comprise hollow fibers, and the applying the pressure may comprise limiting the pressure to a level that does not cause bursting of the fibers of the microfilter membrane.

For any of the methods described herein, the microfilter membrane may comprise a flat sheet membrane, and the applying the pressure may comprise limiting the pressure to a level that does not cause bursting or rupture of the flat sheet membrane of the microfilter membrane.

For any of the methods described herein, the pore size profile may be inversely proportional to the level of the pressure that results in expulsion of the test fluid from pores of the porous microfilter membrane, wherein pressure applied (P), surface tension of the test fluid (*γ*), contact angle between the membrane surface and the test fluid (θ), and diameter of the pore at its narrowest point (D) are related as: [equation I] $P = 4 * γ * \left(cos θ\right) / D$

For any of the methods described herein, determining the pore size profile may comprise using [equation I]: $P = 4 * γ * \left(cos θ\right) / D .$

### BRIEF DESCRIPTION OF THE DRAWINGS

**FIG. 1** is a schematic diagram of a porous microfilter of some embodiments.
**FIG. 2A-2B** are flow diagrams. **FIG. 2A** is a flow diagram depicting methods of determining a pore size profile of a microfilter of some embodiments. **FIG. 2B** is a flow diagram depicting methods of filtering a cell culture product of some embodiments.
FIGs. 3A**-B** are graphs showing ATR-FTIR spectroscopy spectra obtained for fibers and for microfilters according to methods of some embodiments.
FIGs. 4A**-D** are field emission scanning electron microscopy (FE-SEM) micrographs of pore morphology before **(****FIGs. 4A** & **4C****)** and after **(****FIGs. 4B** & **4D****)** storage solution (in this example, glycerol) removal and drying procedure. Images were obtained at 20,000x **(**FIGs. 4A**-B)** and 50,000x **(**FIGs. 4C**-D)** magnification.
**FIG. 5** is a graph showing that measurements of pore size profiles in accordance with embodiments herein were repeatable, with pore size profiles measured in agreement with expected trends.

### DETAILED DESCRIPTION

The distribution of pore sizes of a microfilter is an attribute that impacts the performance of the filter and consequently, impacts the yield of the harvest of therapeutic protein from bioreactors. Variability in pore size profiles from one filter to the next therefore has the potential to significantly alter process performance. Many microfilter products are often provided saturated in a storage solution, also sometimes referred to as a humectant, such as glycerin. It is observed herein that storage solution can interfere with the accuracy and reliability of measurements of pore size profiles. However, conventional approaches for removing storage solutions may disturb the pore structure of the microfilter, impacting microfilter performance. Accordingly, conventional approaches for assessing pore sizes of microfilters may generate inaccuracies, which may lead to the use of filters that have an unsuitable pore size distribution for filtering the cell culture product of a therapeutic protein.

Described herein are methods for determining a pore size profile of a microfilter, and methods for filtering a cell culture product. The methods described herein can accurately ascertain a pore size profile of a microfilter. In the methods, a microfilter may be provided saturated in storage solution. The methods can comprise contacting (e.g., immersing) the microfilter with an intermediate solvent, so as to dissolve the storage solution in the intermediate solvent, thus removing storage solution from the microfilter and desaturating the microfilter so that no storage solution remains (though it is contemplated that trace amounts of storage solution may still be present). The microfilter can optionally be dried after contact with intermediate solvent to further remove residual storage solution and/or intermediate solvent. Then, the pore size profile of the microfilter can be calculated based on a level of pressure that results in the flow of gas or liquid through the microfilter. A test fluid may be applied to the de-saturated microfilter. A pressure may then be applied to a first surface of the de-saturated microfilter, for example by applying a gas or a liquid to the first surface. The pressure may result in the flow of gas or liquid through the microfilter, so that gas or liquid may be detected emanating from a second surface that is on the opposite side of the microfilter from the first surface. The method may comprise increasing the pressure until it results in the flow of gas or liquid through the microfilter. Based on the determined pore size of the microfilter, the microfilter may be selected or rejected for filtering a cell culture product pool comprising therapeutic protein.

As used herein a "pore size profile" refers to a pore size distribution of a microfilter. The pore size profile may be expressed as a mathematical distribution, as a range, or as a single numerical value such as a mean or median, optionally accompanied by characterization of the distribution such as standard deviation or quartile values. As it will be appreciated that a microfilter generally contains numerous pores that are not each exactly the same size, the identification of a pore size profile by one or more numerical values does not imply that every single pore of the microfilter is exactly the same size. For conciseness, a "pore size profile" may be referred to herein as a "pore size." Unless explicitly stated otherwise or clear from context, it will be understood that the "pore size" refers to a pore size profile, rather than an implication that every single pore of the microfilter is the same size. Rigorous characterization of the pore size profile of microfilters may be accomplished by methods described herein, and is advantageous in order to ensure consistent process performance in the applications for which these filters are used.

### Capillary flow porometry (CFP)

Capillary Flow Porometry (CFP) is a well-established analytical technique for measuring pore sizes in hollow fiber (and flat sheet) microfiltration (MF) membranes. The method was described by A. Einstein in 1923 (A. Einstein and H. Mühsam. Deutsche medizinische Wochenschrift. v49, no. 31 (1923):1012-1013).

For any of the methods described herein, the pore size profile may be determined by capillary flow porometry (CFP). In CFP, the membrane (such as a microfilter) is first filled with a test fluid. The membrane is then subjected to increasing gas pressure, and the test fluid, which is initially held in place in the membrane pores by capillary forces, is expelled from the pores. As gas expels the liquid from the pores, gas flow across the membrane is measured. The test fluid is expelled from the pores as a function of the pressure applied (*P*), the surface tension of the test fluid (*y*), the contact angle between the membrane surface and the test fluid (*θ*), and the diameter of the pore at its narrowest point (*D*) according to the Young-Laplace equation: [Equation I] $P = 4 γ cos θ / D$

Accordingly, for methods described herein, wherein the pore size profile may be inversely proportional to the level of the pressure that results in expulsion of the test fluid (and/or emission of gas) from pores of the porous microfilter as described in [Equation I]. In methods of some embodiments, [Equation 1] is used to determine a pore size profile of a microfilter. The determination of pore size profile may be based on a level of the pressure that results in the flow of the gas or liquid from the second surface as described herein.

Performance of CFP may comprise homogeneous wetting of the membrane to be tested with an appropriately selected test fluid, for example a test fluid as described herein.

To remove a storage solution such as glycerol trapped inside of the pores of a membrane (such as HFM) such as in microfilters, a significant amount of mechanical pressure is typically required to create motion in the liquid due to flow resistance caused by high surface tension of the liquid and small pore sizes of the membrane which may be on the order of 5 - 200 nm. A common practice by some membrane suppliers is to utilize a very low surface tension liquid such as Porolfil^{®} product, (or other liquids such as Fluorinert^{®} product, Porefil^{®} product, Porewick^{®} product, or Galwick^{®} product) to "wet" the membrane (fill the pores of the membrane), with the anticipation that glycerol will be automatically displaced or pumped out the membrane by the low surface tension liquid. CFP measurements are then performed directly on the membrane with the presumption of uniform Porofil^{®} product wetting. A low surface tension liquid can wet or fill the pores of a membrane such as HFM when there is no liquid trapped in these pores, since the low surface tension liquid simply displaces air trapped in the pores. The viscosity of air is 1000 times less than that of liquid, so the air flow resistance is negligible compared to liquid. However, a highly wettable, low surface tension liquid cannot displace another liquid inside the pores of a membrane without additional mechanical pressure. This conventional practice typically results in errors and inconsistency of CFP measurement. More delicate procedures, such as in methods described herein, can remove glycerol in a membrane such as HFM or flat sheet prior to CFP measurement and preserve the pore structures.

### Microfilters

As used herein, "microfilters" refer to filter membranes comprising or consisting of flat-sheet membranes (FSM) or hollow fiber membranes (HFM). The microfilter comprises pores, and thus may also be referred to as a "porous microfilter." The membranes may have a specified molecular weight cutoff (MWCO), though rigorous determination of the precise MWCO can be a complex analytical challenge. In the absence of standardized methods for determining MWCO, membrane manufacturers often simply follow a manufacturing procedure according to recipe known to yield membranes in the appropriate MWCO range. The microfilter may comprise or consist of polymers such as polysulfone, polyethersulfone, polyvinylidene fluoride, or cellulose, or combination of two or more of the listed items. In some embodiments, a microfilter is an ultrafiltration (UF) filter. As a microfilter refers to a type of membrane, a "microfilter" may also be referred to herein as a "microfilter membrane." Accordingly, a "porous microfilter" may also be referred to as a "porous microfilter membrane." The membrane may comprise or consist of a HFM or FSM.

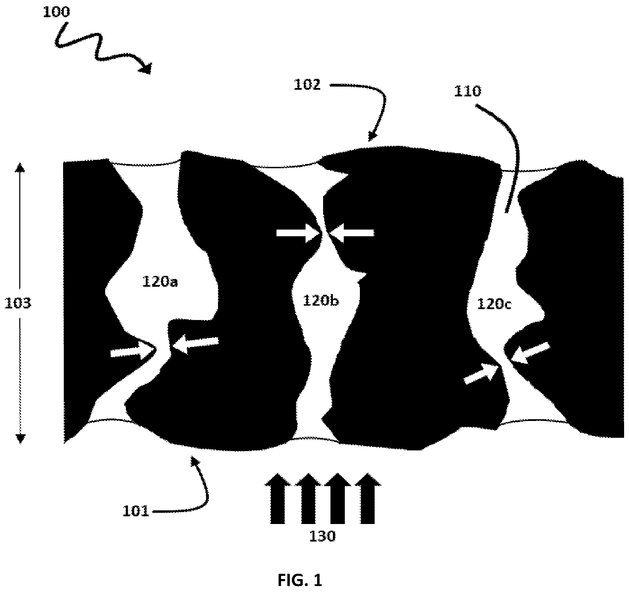

**FIG.** 1 shows a schematic representation of a microfilter membrane **100** comprising a first surface **101** and second surface **102,** filled with a test fluid **110** and containing pore structures **120a, 120b, 120c.** The narrowest points of each pore structure **120a, 120b, 120c** are indicated by white arrows. When pressure is applied (black arrows, 130) to the first surface **101,** the test fluid is emptied toward the second surface **102** from the largest pores first, and as the pressure is increased, pores of successively smaller diameter are subsequently emptied (manifesting as an increase in the flow measured from the second surface). The material between the first surface **101** and the second surface **102** may be referred to as a "matrix" **103.**

A microfilter in which all or substantially all of the storage solution has been removed may be referred to as a "de-saturated" (or "desaturated") microfilter. All or substantially all of the storage solution has been removed when any storage solution in the microfilter is below a limit of detection as measured by Attenuated Total Reflectance-Fourier transform infrared (ATR-FTIR) spectroscopy. That is, the ATR-FTIR spectrum may match that of pure microfilter material alone. For example, if the microfilter is made of polysulfone or polyethersulfone, the ATR-FTIR spectrum of the microfilter may be compared to that to pure polysulfone or polyethersulfone (as appropriate) to determine if any storage solution or other wetting liquid may be detected. A "dry" microfilter as used herein refers to a de-saturated microfilter.

In some embodiments, a microfilter is selected to have a specified MWCO. For example, a microfilter may be selected for use in filtering only if the determined pore size profile of the microfilter is within a specified range, for example accommodating passage of molecules having a molecular weight of up to 50 kDa, 75 kDa, 100 kDa, 150 kDa, 200 kDa, 500 kDa, 750kDa, or 1000 kDa through the microfilter. In some embodiments, a microfilter is selected for use in filtering only if the determined pore size profile of the microfilter accommodates passage of molecules having a molecular weight of less than 50 kDa, 75 kDa, 100 kDa, 150 kDa, 200 kDa, 500 kDa, 750kDa, or 1000 kDa through the microfilter.

In some embodiments, a microfilter is selected to have a pore size profile in which the average diameter of the pores falls within a specified range, for example, 5-120 nanometers, 5-100 nanometers, 60-120 nanometers, or 60-100 nanometers.

### Storage solutions

Microfilters are often provided saturated in storage solutions, which may also be referred to as preservative liquids or humectants. UF HFM or FSM microfilters are typically impregnated to saturation with storage solutions glycerin or other nonvolatile liquid to preserve the pore structure against collapse or other physical deformation during drying of water present in the membrane during fiber manufacturing. Such storage solutions typically have surface tension higher than what is necessary for performance of CFP on UF membranes. Therefore, preparation of microfilter samples for CFP measurements advantageously is by methods as described herein that (A) remove the storage solution and replaces it with the chosen CFP test fluid, and (B) do so without substantially disturbing the pore structure of the membrane. It will be appreciated that the pore structure is not "substantially" disturbed when no perturbations are detected in a sample, such as a sample observed by SEM, and/or when at least 85%, 90%, or 95% of the pores in the sample retain their structure from prior to removal of the storage solution.

In methods of some embodiments, the storage solution comprises or consists of a water-soluble non-volatile solution which is a polyol. For example, the polyol may comprise or consist of glycerin or glycerol.

### Intermediate solvents

Intermediate solvents for methods of some embodiments herein comprise or consist of solvents that 1) dissolve the storage solution, 2) do not dissolve the filter membrane, and 3) can be removed by drying (evaporation) or by displacement with the test fluid.

Advantageously, intermediate solvents may have lower surface tension than water (which is about 70 mN m⁻¹), leading to weaker capillary forces exerted on the pore structure and allowing the evaporation to proceed without deforming the pores. Examples of suitable intermediate solvents for methods herein include alcohol, such as isopropyl alcohol. In methods of some embodiments, the intermediate solvent comprises or consists of solvents that 1) dissolve the storage solution, 2) do not dissolve the filter membrane, and 3) can be removed by drying (evaporation) or by displacement with the test fluid.

### Test Fluids

Test fluids suitable for methods herein include fluids that are capable of completely wetting the surface of the microfilter. Typically, fluids that have a low or zero contact angle with the filter matrix material are generally capable of completely wetting the matrix of the microfilter. Accordingly, for methods described herein, a contact angle of the test fluid to a surface (e.g., a first surface as described herein) may be low enough to saturate the microfilter with test fluid. For example, the contact angle of the test fluid may be no more than 15° or no more than 10°, or may be 0°.

It can be advantageous for test fluids of methods described herein to have a low surface tension. For small pore sizes typical of UF membranes, the pressures to complete CFP measurement may be higher than the burst pressure of the membrane (HFM or FSM). Accordingly, a test fluid with a low surface tension may permit CFP measurements to be performed at low applied pressures, thus avoiding bursting of the membrane. By way of example, the test fluid may have a lower surface tension than water. The surface tension of water may be referenced as 70 mN m⁻¹. Accordingly, the test fluid may have a surface tension less than 70 mN m⁻¹.

Other advantageous characteristics of test fluids suitable for methods described herein include test fluids that do not dissolve, swell, and/or otherwise interact with the membrane material of construction for the microfilter.

Test fluids for methods described herein may comprise or consist of organic solvents or mixtures thereof. In methods of some embodiments, the test fluid comprises or consists of Porofil^{®} product, Fluorinert^{®} product, Porefil^{®} product, Porewick^{®} product, or Galwick^{®} product.

### Therapeutic Proteins

As used herein "therapeutic protein" and variations of this root term has its ordinary and customary meaning as would be understood by one of ordinary skill in the art in view of this disclosure. It refers to a polypeptide for medical use in a subject, such as a human subject, or for veterinary use in a non-human mammal. A therapeutic protein may be a protein for medical use, such as a candidate for medical use, or a protein approved for medical use by a government authority such as the FDA or EMA.

In methods described herein, the therapeutic protein may be selected from the group consisting of: an antibody, an antigen binding protein, an antibody protein product, a Bi-specific T cell engager (BiTE^{®}) molecule, a multispecific antibody (such as a bispecific antibody or trispecific antibody), an Fc fusion protein, a recombinant protein, a synthetic peptide, and an active fragment of a recombinant protein.

An "antibody" has its customary and ordinary meaning as understood by one of ordinary skill in the art in view of this disclosure. It refers to an immunoglobulin with specific binding to the target antigen, and includes, for instance, chimeric, humanized, and fully human antibodies. By way of example, the antibody may be a monoclonal antibody. By way of example, human antibodies can be of a specified isotype, including IgG (including IgG1, IgG2, IgG3 and IgG4 subtypes), IgA (including IgA1 and IgA2 subtypes), IgM and IgE. A human IgG antibody generally comprises two full-length heavy chains and two full-length light chains. Antibodies may be derived solely from a single source, or may be "chimeric," that is, different portions of the antibody may be derived from two or more different antibodies from the same or different species. It will be understood that once an antibody is obtained from a source, it may undergo further engineering, for example to enhance stability and folding. Accordingly, it will be understood that a "human" antibody may be obtained from a source, and may undergo further engineering, for example in the Fc region. The engineered antibody may still be referred to as a type of human antibody. Similarly, variants of a human antibody, for example those that have undergone affinity maturation, will also be understood to be "human antibodies" unless stated otherwise. In some embodiments, an antibody comprises, consists essentially of, or consists of a human, humanized, or chimeric monoclonal antibody.

A "heavy chain" of an antibody, antigen binding protein, antibody protein product, Bi-specific T cell engager molecule, or multispecific antibody includes a variable region ("VH"), and three constant regions: CH1, CH2, and CH3. A "light chain" of an antibody, antigen binding protein, antibody protein product, Bi-specific T cell engager molecule, or multispecific antibody includes a variable region ("VL"), and a constant region ("CL"). Human light chains include kappa chains and lambda chains. Example light chain constant regions suitable for antigen binding proteins include human lambda and human kappa constant regions.

In various aspects, the therapeutic protein is an antibody protein product. As used herein, the term "antibody protein product" refers to any one of several antibody alternatives which in various instances is based on the architecture of an antibody but is not found in nature. In some aspects, the antibody protein product has a molecular-weight within the range of at least about 12 KDa to about 250 kDa. In certain aspects, the antibody protein product has a valency (n) range from monomeric (n = 1), to dimeric (n = 2), to trimeric (n = 3), to tetrameric (n = 4), if not higher order valency. Antibody protein products in some aspects are those based on the full antibody structure and/or those that mimic antibody fragments which retain full antigen-binding capacity, e.g., scFvs, Fabs and VHH/VH (discussed below). The smallest antigen binding antibody fragment that retains its complete antigen binding site is the Fv fragment, which consists entirely of variable (V) regions. A soluble, flexible amino acid peptide linker is used to connect the V regions to a scFv (single chain fragment variable) fragment for stabilization of the molecule, or the constant (C) domains are added to the V regions to generate a Fab fragment [fragment, antigen-binding]. Both scFv and Fab fragments can be easily produced in host cells, e.g., prokaryotic host cells. Other antibody protein products include disulfide-bond stabilized scFv (ds-scFv), single chain Fab (scFab), as well as di- and multimeric antibody formats like dia-, tria- and tetra-bodies, or minibodies (miniAbs) that comprise different formats consisting of scFvs linked to oligomerization domains. The smallest fragments are VHH/VH of camelid heavy chain Abs as well as single domain Abs (sdAb). The building block that is most frequently used to create novel antibody formats is the single-chain variable (V)-domain antibody fragment (scFv), which comprises V domains from the heavy and light chain (VH and VL domain) linked by a peptide linker of ~15 amino acid residues. A peptibody or peptide-Fc fusion is yet another antibody protein product. The structure of a peptibody consists of a biologically active peptide grafted onto an Fc domain. Peptibodies are well-described in the art. See, e.g., Shimamoto et al.., mAbs 4(5): 586-591 (2012). Bispecific T-cell engager molecules, for example those comprising a half-life extension moiety are also examples of antibody protein products.

Therapeutic proteins suitable for the methods described herein can include polypeptides, including those that bind to one or more of the following: CD proteins, including CD3, CD4, CD8, CD19, CD20, CD22, CD30, and CD34; including those that interfere with receptor binding. HER receptor family proteins, including HER2, HER3, HER4, and the EGF receptor. Cell adhesion molecules, for example, LFA-I, Mol, pl50, 95, VLA-4, ICAM-I, VCAM, and alpha v/beta 3 integrin. Growth factors, such as vascular endothelial growth factor ("VEGF"), growth hormone, thyroid stimulating hormone, follicle stimulating hormone, luteinizing hormone, growth hormone releasing factor, parathyroid hormone, Mullerian-inhibiting substance, human macrophage inflammatory protein (MIP-1alpha), erythropoietin (EPO), nerve growth factor, such as NGF-beta, platelet-derived growth factor (PDGF), fibroblast growth factors, including, for instance, aFGF and bFGF, epidermal growth factor (EGF), transforming growth factors (TGF), including, among others, TGF- α and TGF-β, including TGF-β1, TGF-β2, TGF-β3, TGF- β4, or TGF- β5, insulin-like growth factors-I and -II (IGF-I and IGF-II), des(I-3)-IGF-I (brain IGF-I), and osteoinductive factors. Insulins and insulin-related proteins, including insulin, insulin A-chain, insulin B-chain, proinsulin, and insulin-like growth factor binding proteins. Coagulation and coagulation-related proteins, such as, among others, factor VIII, tissue factor, von Willebrands factor, protein C, alpha-1-antitrypsin, plasminogen activators, such as urokinase and tissue plasminogen activator ("t-PA"), bombazine, thrombin, and thrombopoietin; (vii) other blood and serum proteins, including but not limited to albumin, IgE, and blood group antigens. Colony stimulating factors and receptors thereof, including the following, among others, M-CSF, GM-CSF, and G-CSF, and receptors thereof, such as CSF-1 receptor (c-fms). Receptors and receptor-associated proteins, including, for example, flk2/flt3 receptor, obesity (OB) receptor, LDL receptor, growth hormone receptors, thrombopoietin receptors ("TPO-R," "c-mpl"), glucagon receptors, interleukin receptors, interferon receptors, T-cell receptors, stem cell factor receptors, such as c-Kit, and other receptors. Receptor ligands, including, for example, OX40L, the ligand for the OX40 receptor. Neurotrophic factors, including bone-derived neurotrophic factor (BDNF) and neurotrophin-3, -4, -5, or -6 (NT-3, NT-4, NT-5, or NT-6). Relaxin A-chain, relaxin B-chain, and prorelaxin; interferons and interferon receptors, including for example, interferon-α, -β, and -γ, and their receptors. Interleukins and interleukin receptors, including IL-I to IL-33 and IL-I to IL-33 receptors, such as the IL-8 receptor, among others. Viral antigens, including an AIDS envelope viral antigen. Lipoproteins, calcitonin, glucagon, atrial natriuretic factor, lung surfactant, tumor necrosis factor-alpha and -beta, enkephalinase, RANTES (regulated on activation normally T-cell expressed and secreted), mouse gonadotropin-associated peptide, DNAse, inhibin, and activin. Integrin, protein A or D, rheumatoid factors, immunotoxins, bone morphogenetic protein (BMP), superoxide dismutase, surface membrane proteins, decay accelerating factor (DAF), HIV envelope, transport proteins, homing receptors, addressins, regulatory proteins, immunoadhesins, antibodies. Myostatins, TALL proteins, including TALL-I, amyloid proteins, including but not limited to amyloid-beta proteins, thymic stromal lymphopoietins ("TSLP"), RANK ligand ("RANKL" or "OPGL"), c-kit, TNF receptors, including TNF Receptor Type 1, TRAIL-R2, angiopoietins, and biologically active fragments or analogs or variants of any of the foregoing.

Examples of therapeutic proteins suitable for the methods described herein include antibodies or variants thereof comprising an IgG1 constant region comprising one or more of the following mutations numbered according to the EU system and selected from the group consisting of: L242C, A287C, R292C, N297G, V302C, L306C, and K334C, such as infliximab, bevacizumab, cetuximab, ranibizumab, palivizumab, abagovomab, abciximab, actoxumab, adalimumab, afelimomab, afutuzumab, alacizumab, alacizumab pegol, ald518, alemtuzumab, alirocumab, altumomab, amatuximab, anatumomab mafenatox, anrukinzumab, apolizumab, arcitumomab, aselizumab, altinumab, atlizumab, atorolimiumab, tocilizumab, bapineuzumab, basiliximab, bavituximab, bectumomab, belimumab, benralizumab, bertilimumab, besilesomab, bevacizumab, bezlotoxumab, biciromab, bivatuzumab, bivatuzumab mertansine, blinatumomab, blosozumab, brentuximab vedotin, briakinumab, brodalumab, canakinumab, cantuzumab mertansine, cantuzumab mertansine, caplacizumab, capromab pendetide, carlumab, catumaxomab, cc49, cedelizumab, certolizumab pegol, cetuximab, citatuzumab bogatox, cixutumumab, clazakizumab, clenoliximab, clivatuzumab tetraxetan, conatumumab, crenezumab, cr6261, dacetuzumab, daclizumab, dalotuzumab, daratumumab, demcizumab, denosumab, detumomab, dorlimomab aritox, drozitumab, duligotumab, dupilumab, ecromeximab, eculizumab, edobacomab, edrecolomab, efalizumab, efungumab, elotuzumab, elsilimomab, enavatuzumab, enlimomab pegol, enokizumab, enoticumab, ensituximab, epitumomab cituxetan, epratuzumab, erenumab, erlizumab, ertumaxomab, etaracizumab, etrolizumab, evolocumab, exbivirumab, fanolesomab, faralimomab, farletuzumab, fasinumab, fbta05, felvizumab, fezakinumab, ficlatuzumab, figitumumab, flanvotumab, fontolizumab, foralumab, foravirumab, fresolimumab, fulranumab, futuximab, galiximab, ganitumab, gantenerumab, gavilimomab, gemtuzumab ozogamicin, gevokizumab, girentuximab, glembatumumab vedotin, golimumab, gomiliximab, gs6624, ibalizumab, ibritumomab tiuxetan, icrucumab, igovomab, imciromab, imgatuzumab, inclacumab, indatuximab ravtansine, infliximab, intetumumab, inolimomab, inotuzumab ozogamicin, ipilimumab, iratumumab, itolizumab, ixekizumab, keliximab, labetuzumab, lebrikizumab, lemalesomab, lerdelimumab, lexatumumab, libivirumab, ligelizumab, lintuzumab, lirilumab, lorvotuzumab mertansine, lucatumumab, lumiliximab, mapatumumab, maslimomab, mavrilimumab, matuzumab, mepolizumab, metelimumab, milatuzumab, minretumomab, mitumomab, mogamulizumab, morolimumab, motavizumab, moxetumomab pasudotox, muromonab-cd3, nacolomab tafenatox, namilumab, naptumomab estafenatox, narnatumab, natalizumab, nebacumab, necitumumab, nerelimomab, nesvacumab, nimotuzumab, nivolumab, nofetumomab merpentan, ocaratuzumab, ocrelizumab, odulimomab, ofatumumab, olaratumab, olokizumab, omalizumab, onartuzumab, oportuzumab monatox, oregovomab, orticumab, otelixizumab, oxelumab, ozanezumab, ozoralizumab, pagibaximab, palivizumab, panitumumab, panobacumab, parsatuzumab, pascolizumab, pateclizumab, patritumab, pemtumomab, perakizumab, pertuzumab, pexelizumab, pidilizumab, pintumomab, placulumab, ponezumab, priliximab, pritumumab, PRO 140, quilizumab, racotumomab, radretumab, rafivirumab, ramucirumab, ranibizumab, raxibacumab, regavirumab, reslizumab, rilotumumab, rituximab, robatumumab, roledumab, romosozumab, rontalizumab, rovelizumab, ruplizumab, samalizumab, sarilumab, satumomab pendetide, secukinumab, sevirumab, sibrotuzumab, sifalimumab, siltuximab, simtuzumab, siplizumab, sirukumab, solanezumab, solitomab, sonepcizumab, sontuzumab, stamulumab, sulesomab, suvizumab, tabalumab, tacatuzumab tetraxetan, tadocizumab, talizumab, tanezumab, taplitumomab paptox, tefibazumab, telimomab aritox, tenatumomab, tefibazumab, teneliximab, teplizumab, teprotumumab, TGN1412, tremelimumab, ticilimumab, tildrakizumab, tigatuzumab, TNX-650, tocilizumab, toralizumab, tositumomab, tralokinumab, trastuzumab, TRBS07, tregalizumab, tucotuzumab celmoleukin, tuvirumab, ublituximab, urelumab, urtoxazumab, ustekinumab, vapaliximab, vatelizumab, vedolizumab, veltuzumab, vepalimomab, vesencumab, visilizumab, volociximab, vorsetuzumab mafodotin, votumumab, zalutumumab, zanolimumab, zatuximab, ziralimumab, or zolimomab aritox.

In some embodiments, the therapeutic protein is a BiTE^{®} molecule. BiTE^{®} molecules are engineered bispecific antigen binding constructs which direct the cytotoxic activity of T cells against cancer cells. They are the fusion of two single-chain variable fragments (scFvs) of different antibodies, or amino acid sequences from four different genes, on a single peptide chain of about 55 kDa. One of the scFvs binds to T cells via the CD3 receptor, and the other to a tumor cell via a tumor specific molecule. Blinatumomab (BLINCYTO^{®} product) is an example of a BiTE^{®} molecule, specific for CD19. BiTE^{®} molecules that are modified, such as those modified to extend their half-lives, can also be used in the disclosed methods. In various aspects, the polypeptide is an antigen binding protein, e.g., a BiTE^{®} molecule. In some embodiments, an antibody protein product comprises a BiTE^{®} molecule.

### Cell cultures and cell culture products

It will be appreciated that therapeutic proteins described herein may be produced by cell culture, and thus may be comprised by cell culture products. Cell culture products that may be filtered in accordance with methods herein include those of a cell culture selected from the group consisting of: mammalian cells such as Chinese Hamster Ovary (CHO) cells, HeLa cells, baby hamster kidney (BHK) cells, monkey kidney cells (COS), human hepatocellular carcinoma cells (e.g., Hep G2), or human epithelial kidney 293 cells; insect cells, such as Sf21/Sf9 or *Trichoplusia* ni Bti-Tn5bl-4 cells; yeast cells, such as *Saccharomyces* or *Pichia* cells; plant cells; avian cells such as chicken cells; and prokaryotic cells such as *Escherichia coli* cells.

### Methods of determining pore size profiles

In some embodiments, methods of determining pore size profiles are described. The method may comprise providing a saturated porous microfilter. The porous microfilter may comprise a first surface, a second surface, and a matrix disposed between the first and the second surface. The matrix may be saturated by a storage solution. The method may further comprise contacting the saturated porous microfilter with an intermediate solvent, so that the storage solution dissolves in the intermediate solvent, thus de-saturating the microfilter. The method may further comprise applying a test fluid to the de-saturated microfilter, thus re-saturating the microfilter. The method may further comprise applying a pressure to the first surface of the re-saturated microfilter. The pressure may be applied by contacting the first surface with a gas or liquid. The method may further comprise detecting a flow of the gas, liquid, and/or test fluid from the second surface in response to said pressure. The method may further comprise determining the pore size profile of the microfilter based on a level of the pressure that results in the flow of the gas, liquid, and/or test fluid from the second surface.

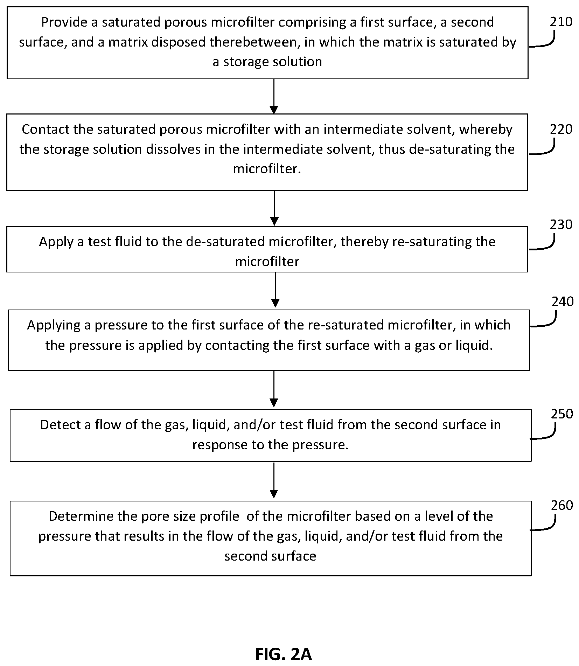

A method of determining a pore size profile according to some embodiments is illustrated in **FIG. 2A****.** A saturated porous microfilter is provided, comprising a first surface, a second surface, and a matrix disposed therebetween, in which the matrix is saturated by a storage solution **210.** The saturated porous microfilter is contacted with an intermediate solvent, in which the storage solution dissolves in the intermediate solvent, thus de-saturating the microfilter **220.** For example, contacting the microfilter with intermediate solvent may comprise immersing the microfilter in intermediate solvent. Then, a test fluid is applied to the de-saturated microfilter, thus re-saturating the microfilter **230.** A pressure is applied to the first surface of the re-saturated microfilter **240.** The pressure may be applied by contacting the first surface with a gas or a liquid. After the pressure is applied, a flow of the gas, liquid, and/or test fluid from the second surface in response to the pressure is detected **250.** For example, expulsion from the second surface of the test fluid itself, or of the gas and/or liquid used to apply pressure may be detected. The pressure may be increased gradually (e.g., continuously or stepwise) until the flow of the gas, liquid, and/or test fluid from the second surface is detected. Once the flow of the gas, liquid, and/or test fluid from the second surface is detected, the pore size profile of the microfilter may be determined based on a level of the pressure that results in the flow of the gas, liquid, and/or test fluid from the second surface **260.** The pore size profile of the microfilter may be determined. For example, the pore size profile may be determined by CFP as described herein. For example, Equation 1 may be used to determine the pore size profile based on the level of pressure. In some embodiments, the microfilter may be selected for use in filtering only if the determined pore size profile of the microfilter is within a specified range. The specified range may be a range that accommodates passage of a therapeutic protein through the porous microfilter. For example, the therapeutic protein may be a specified therapeutic protein as described herein. The method may further comprise filtering the cell culture product comprising the therapeutic protein through the selected microfilter (or a microfilter of the same batch as the selected microfilter). It will be understood that one or more of the noted portions of the method may be repeated, or as appropriate in context, omitted or performed in a difference sequence. In some embodiments, the method comprises drying the de-saturated microfilter prior to applying the test fluid **230.** The drying may be performed until the intermediate solvent is below a limit of detection by ATR-FTIR spectroscopy.

In the methods of some embodiments, the method is performed on a microfilter sample pulled from the HFM manufacturing line before the storage solution is introduced. For such methods it will be appreciated that 210 and 220 may be omitted.

### Methods of filtering a cell culture product

In some embodiments, a method of filtering a cell culture product comprising therapeutic protein is described. The method can comprise providing a saturated porous microfilter comprising a first surface, a second surface, and a matrix disposed therebetween, and in which the matrix is saturated by a storage solution. The method can comprise contacting the saturated porous microfilter with an intermediate solvent, so that the storage solution dissolves in the intermediate solvent, thus de-saturating the microfilter. The method can comprise applying a test fluid to the de-saturated microfilter, thereby re-saturating the microfilter. The method can comprise applying a pressure to the first surface of the re-saturated microfilter, in which the pressure is applied by contacting the first surface with a gas or liquid. The method can comprise detecting a flow of the gas, liquid, and/or test fluid from the second surface in response to said pressure, in which the pore size profile of the microfilter is determined based on a level of the pressure that results in the flow of the gas, liquid, and/or test fluid from the second surface. The microfilter may be selected for use in filtering only if the determined pore size profile of the microfilter is within a specified range that accommodates passage of the therapeutic protein through the porous microfilter. The method may further comprise filtering the cell culture product comprising the therapeutic protein through the selected microfilter. The cell culture product may be the product of a cell culture described herein. It will be appreciated that filtering the cell culture product through the selected microfilter does not require filtering the cell culture through the exact piece of material that was processed according to the method, and also encompasses filtering the cell culture product through a microfilter of the same batch as the microfilter that was tested.

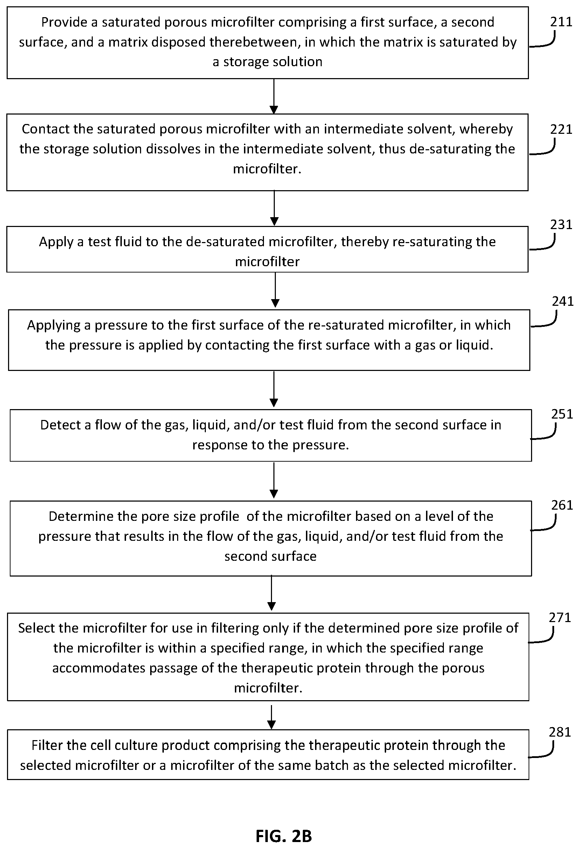

A method of filtering a cell culture product according to some embodiments is illustrated in **FIG. 2B**. A saturated porous microfilter is provided, comprising a first surface, a second surface, and a matrix disposed therebetween, in which the matrix is saturated by a storage solution **211.** The saturated porous microfilter is contacted with an intermediate solvent, in which the storage solution dissolves in the intermediate solvent, thus de-saturating the microfilter **221.** For example, contacting the microfilter with intermediate solvent may comprise immersing the microfilter in intermediate solvent. Then, a test fluid is applied to the de-saturated microfilter, thus re-saturating the microfilter **231.** A pressure is applied to the first surface of the re-saturated microfilter **241.** The pressure may be applied by contacting the first surface with a gas or a liquid. After the pressure is applied, a flow of the gas, liquid, and/or test fluid from the second surface in response to the pressure is detected **251.** For example, expulsion from the second surface of the test fluid itself, or of the gas and/or liquid used to apply pressure may be detected. The pressure may be increased gradually (e.g., continuously or stepwise) until the flow of the gas, liquid, and/or test fluid from the second surface is detected. Once the flow of the gas, liquid, and/or test fluid from the second surface is detected, the pore size profile of the microfilter may be determined based on a level of the pressure that results in the flow of the gas, liquid, and/or test fluid from the second surface **261.** The pore size profile of the microfilter may be determined. For example, the pore size profile may be determined by CFP as described herein. For example, Equation 1 may be used to determine the pore size profile based on the level of pressure. The microfilter may be selected for use in filtering only if the determined pore size profile of the microfilter is within a specified range **271.** The specified range may accommodate passage of the therapeutic protein through the porous microfilter. The method may further comprise filtering the cell culture product comprising the therapeutic protein through the selected microfilter or a microfilter of the same batch as the selected microfilter **281.** It will be understood that the microfilter for which the pore size profile is determined may be a sample of a larger microfilter, and that portions of the microfilter not actually used in the determination of the pore size profile, or other microfilters from the same batch may be used for filtering a cell culture product, and be understood to have a pore size profile in line with the microfilter that is actually tested. It will be understood that one or more of the noted portions of the method may be repeated, or as appropriate in context, omitted or performed in a difference sequence. The cell culture product may be the product of a cell culture described herein. In some embodiments, the method comprises drying the de-saturated microfilter prior to applying the test fluid **231.** The drying may be performed until the intermediate solvent is below a limit of detection by ATR-FTIR spectroscopy.

In the method of filtering a cell culture product of some embodiments, the specified range accommodates passage of molecules having a molecular weight of up to 50 kDa, 75 kDa, 100 kDa, 150 kDa, 200 kDa, 500 kDa, 750kDa, or 1000 kDa through the porous microfilter. In the method of filtering a cell culture product of some embodiments, the specified range is an average pore diameter of 5-120 nanometers, or 60-100 nanometers, 5-100 nanometers, or 60-120 nanometers.

In accordance with method of filtering a cell culture product of some embodiments, the cell culture product may comprise cells and/or components of cells in addition to the therapeutic protein. For example, the cell culture product may comprise cell debris and/or host cell protein in addition to the therapeutic protein. The filtering may separate the therapeutic protein from some or all of the other substances in the cell culture product by permitting the therapeutic protein to pass through the microfilter, while other portions of the cell culture product do not.

Examples of suitable cell culture products includes that of a cell culture selected from the group consisting of: mammalian cells such as Chinese Hamster Ovary (CHO) cells, HeLa cells, baby hamster kidney (BHK) cells, monkey kidney cells (COS), human hepatocellular carcinoma cells (e.g., Hep G2), or human epithelial kidney 293 cells; insect cells, such as Sf21/Sf9 or *Trichoplusia* ni Bti-Tn5bl-4 cells; yeast cells, such as *Saccharomyces* or *Pichia* cells; plant cells; avian cells such as chicken cells; and prokaryotic cells such as *Escherichia coli* cells.

Methods of filtering a cell culture product described herein may be used to filter cell culture products comprising a therapeutic protein that is a candidate for medical use, or is approved for medical use by a government authority such as the FDA or EMA. In methods of filtering a cell culture product of some embodiments, the therapeutic protein is selected from the group consisting of: an antibody, an antigen-binding antibody fragment, an antibody protein product, a Bi-specific T cell engager (BiTE^{®}) molecule, a multispecific antibody, an Fc fusion protein, a recombinant protein, and an active fragment of a recombinant protein.

### Additional options for methods described herein

It will be appreciated that for methods described herein, a sample may be tested from a batch of microfilters to determine whether the microfilters of the batch may be used to filter cell culture products comprising therapeutic protein. A sample or samples of membrane may also be tested directly, prior to assembly of membrane into a filter device. It will be appreciated that for any methods described herein, the microfilter tested in the methods does not need to be a full-size microfilter, but may also be a sample of a larger microfilter (so that the larger microfilter, and/or other microfilters of the same batch may subsequently be used to filter cell culture products as described herein). In some methods described herein, the method comprises, prior to contacting the saturated microfilter with the intermediate solvent, cutting fibers of the porous microfilter to a specified dimension. The specified dimension may be, for example, a length of 5-10 cm, 5-15 cm, 5-20cm, 10-15 cm, or 10-20cm, or may be a specified length and width (e.g., flat sheet filters may be cut to a specified length and width, such as 10 cm x 10 cm, 10 cm x 20 cm, or 20 cm x 20 cm).

In some methods described herein, the microfilter may be provided in a storage solution, for example glycerin. The method can comprise extracting the storage solution from the microfilter using an intermediate solvent miscible with glycerin but that does not dissolve or excessively deform the membrane. This may be accomplished simply by immersion of the membrane in the solvent. The method can comprise removing the microfilter from the intermediate solvent and drying the microfilter. The drying can allow the intermediate solvent to evaporate. The intermediate solvent may have lower surface tension than water. The microfilter, now free of any liquid, can now be easily contacted with test fluid by simply immersing the microfilter in the test fluid, allowing the test fluid to infiltrate the microfilter. Optionally, the microfilter can be contacted with test fluid before or after mounting the microfilter in a specialized sample holder for CFP.

For any of the methods described herein, the microfilter may be an ultrafiltration membrane or portion thereof. In these methods, the microfilter may comprise, consist essentially of, or consist of polysulfone, polyethersulfone, polyvinylidene fluoride, or cellulose.

For any of the methods described herein, the storage solution may be soluble in the intermediate solvent, in which the intermediate solvent does not dissolve the microfilter, and in which the intermediate solvent vaporizes at 1 atm pressure and 20° C. For any of the methods described herein, the storage solution comprises or consists of a water-soluble non-volatile solution which is a polyol. For example, the polyol may comprise or consist of glycerin or glycerol.

For any of the methods described herein, the intermediate solvent may comprise or consist of an alcohol such as isopropyl alcohol.

For any of the methods described herein, the microfilter may be desaturated when the storage solution in the microfilter (or a sample thereof) is below a limit of detection by ATR-FTIR spectroscopy. When an ATR-FTIR spectrum of the first and/or second surface matches a reference spectrum of pure material of which the microfilter is made (such as polysulfone or polyethersulfone), it may be concluded that the microfilter is de-saturated.

While it is contemplated that sufficient storage solution may be dissolved by the intermediate solvent to permit determination of the pore size profile without further processing, it is further contemplated that further amounts of storage solution and/or intermediate solvent may be removed by drying the de-saturated microfilter prior to applying the test fluid. Accordingly, in some embodiments, a method as described herein further comprises drying the de-saturated microfilter prior to applying the test fluid. In the method of some embodiments, the drying is performed until the intermediate solvent is below a limit of detection by ATR-FTIR spectroscopy. In the method of some embodiments, wherein the drying is performed until the amount of storage solution in the matrix of the microfilter is no more than 1%, 0.5%, 0.1%, or 0.01% of saturation.

For any of the methods described herein, the microfilter comprises hollow fibers membranes or flat sheet membranes, and applying the pressure comprises limiting the pressure to a level that does not cause bursting of the fibers or sheets of the microfilter. As discussed herein, test fluids with low surface tensions, such as less than 70 mN m-¹ may permit CFP to be completed with relatively low pressure, thus avoiding bursting of the fibers or sheets.

### EXAMPLES

### EXAMPLE 1: Sample preparation

UF HFM fiber samples were obtained from Cytiva (fiber type 750E, with 750kDa MWCO). Fibers were cut to the desired length (12 cm) using microshears. The cut segments were placed in appropriate containers which were filled with a solvent (isopropyl alcohol) to remove the preservative liquid (glycerin). The container with the segment and extraction solvent was subject to agitation (orbital shaker, 200 rpm, one hour). The segments were removed from the solvent and allowed to dry (one hour). The segment (referred to as "dry" in the figures and description herein, though they may also) was then ready for introduction of the CFP test fluid (Porofil^{®}) into the membrane, which was accomplished simply by immersing in Porofil.

Attenuated total reflectance-Fourier transform infrared (ATR-FTIR) spectroscopy and field-emission scanning electron microscopy (FE-SEM) measurements were performed at various stages of the sample preparation.

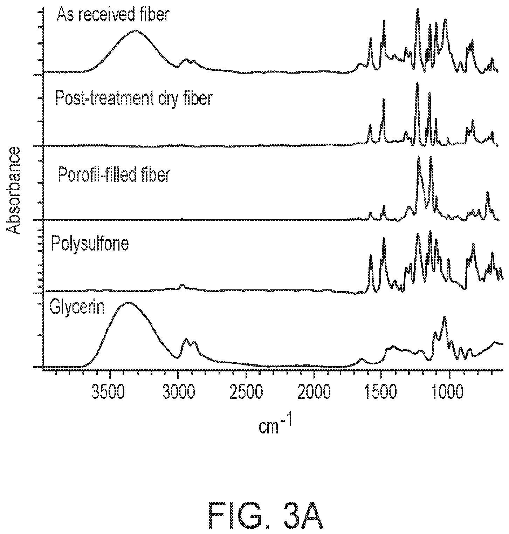

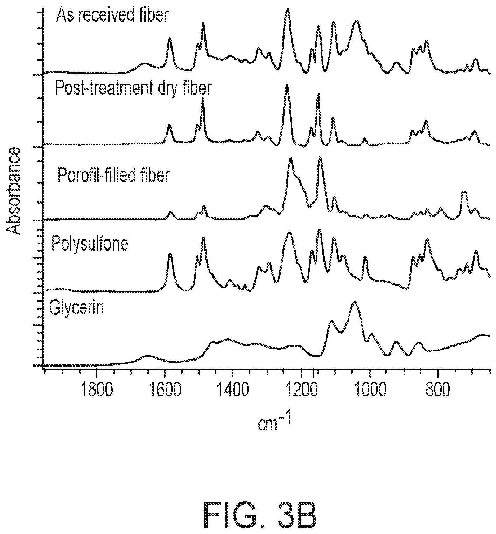

Removal of the storage solution and replacement with the chosen test fluid was demonstrated with ATR-FTIR spectroscopy performed on the inner surface of the HFM samples. FIGs. 3A**-B** show spectra obtained for the as-received fiber (in which glycerin can be readily detected), the "bone dry" fiber (in which no glycerin can be detected, and for which the spectrum matches a reference spectrum of pure Polysulfone, the polymer used to make the HFM), and the fiber which has been backfilled with Porofil^{®} product, a mixture of fluorocarbons (in which Porofil^{®} product can indeed be detected). Reference spectra are included for Glycerin, and Polysulfone.

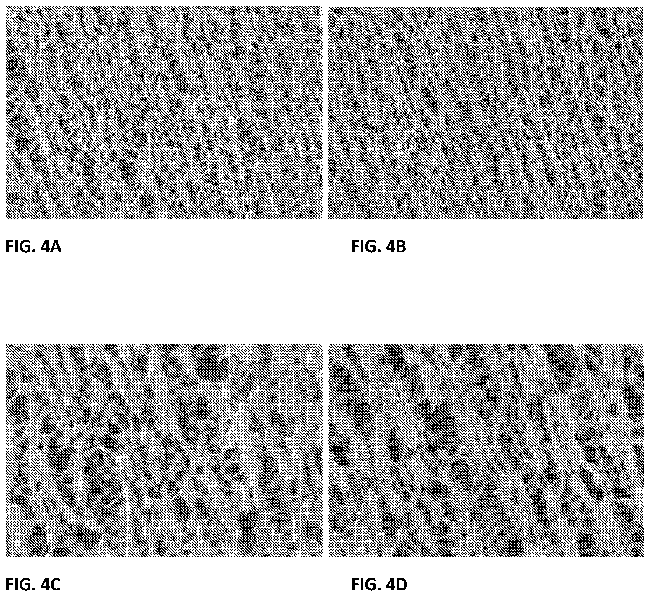

Attempts to directly displace glycerin with Porofil^{®} product (i.e. by soaking as-received fiber in Porofil^{®} product) did not result in removal of glycerol. Spectra of fibers prepared in this manner still showed the presence of large amounts of glycerol. (data not shown) To demonstrate that the sample preparation procedure avoids of significantly disturbing the pore structure of the membrane, high resolution SEM images were obtained from the HFM inner surfaces prior to glycerin removal **(****FIGs. 4A** & **4C****)** and after achieving the "bone dry" state **(****FIGs. 4B** & **4D****).** Representative images are presented in FIGs. 4A**-D,** showing no evidence of damage to the HFM pore structure. FIGs. 4A**-B** depict images obtained at 20,000x magnification, and **FIGs. C-D** depict images obtained at 50,000x magnification.

Accordingly, it was concluded that the methods herein removed all or substantially all storage solution from a microfilter without substantially perturbing the pore size profile, thus rendering the microfilter suitable for CFP.

### EXAMPLE 2: CFP measurements on UF HFM samples prepared

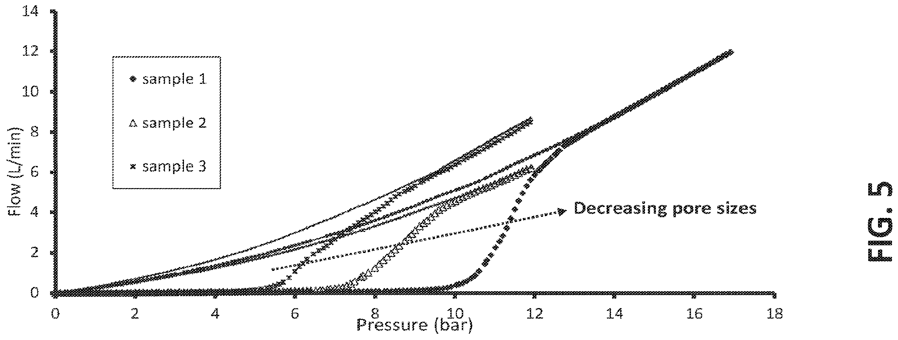

CFP measurements were performed on HFM samples after preparation of these samples according to the procedure in Example 1. The results obtained show repeatable measurements, with pore sizes measured in agreement with expected trends, as shown in **FIG. 5****.** This result demonstrates the effectiveness of the sample preparation procedure described herein.

## Claims

1. A method of determining a pore size profile of a microfilter, the method comprising:
providing (210) a saturated porous microfilter membrane comprising a first surface, a second surface, and a matrix disposed therebetween, wherein the matrix is saturated by a storage solution, wherein the storage solution comprises or consists of a water soluble non-volatile solution which is a polyol, preferably glycerin;
contacting (220) the saturated porous microfilter membrane by immersing in an intermediate solvent, whereby the storage solution dissolves in the intermediate solvent, thereby de-saturating the membrane;
applying (230) a test fluid to the de-saturated membrane, thereby re-saturating the membrane;
applying (240) a pressure to the first surface of the re-saturated membrane, wherein the pressure is applied by contacting the first surface with a gas or liquid;
detecting (250) a flow of the gas, liquid, and/or test fluid from the second surface in response to said pressure; and
determining (260) the pore size profile of the porous microfilter membrane based on a level of the pressure that results in the flow of the gas, liquid, and/or test fluid from the second surface,
wherein de-saturating the microfilter membrane comprises the storage solution being below a limit of detection by ATR-FTIR spectroscopy.

2. A method of filtering a cell culture product comprising a therapeutic protein, the method comprising the method of determining a pore size profile of a microfilter according to claim 1 and further:
selecting (271) the membrane for use in filtering only if the determined pore size profile of the membrane is within a specified range, wherein the specified range accommodates passage of the therapeutic protein through the porous microfilter membrane; and
filtering (281) the cell culture product comprising the therapeutic protein through the selected microfilter membrane or a microfilter membrane of the same batch as the selected microfilter membrane.

3. The method of claim 2, wherein the specified range accommodates passage of molecules having a molecular weight of up to 750kDa through the porous microfilter membrane.

4. The method of claim 2 or 3, wherein the specified range is 5-120 nanometers.

5. The method of any one of claims 2-4, wherein the therapeutic protein is selected from the group consisting of: an antibody, an antigen-binding antibody fragment, an antibody protein product, a Bi-specific T cell engager (BiTE^{®}) molecule, a multispecific antibody, an Fc fusion protein, a recombinant protein, and an active fragment of a recombinant protein.

6. The method of any one of the preceding claims, wherein the microfilter membrane comprises polysulfone, polyethersulfone, polyvinylidene fluoride, or cellulose.

7. The method of claim 1 or 2, wherein the polyol comprises glycerol, such as glycerin.

8. The method of any one of the preceding claims, wherein the intermediate solvent comprises an alcohol, such as isopropyl alcohol.

9. The method of any one of the preceding claims, further comprising drying the de-saturated microfilter membrane prior to applying the test fluid.

10. The method of any one of the preceding claims, wherein de-saturating the microfilter membrane comprises the storage solution being below a limit of detection by ATR-FTIR spectroscopy, wherein an ATR-FTIR spectrum of the first and/or second surface matches a reference spectrum of pure material of which the microfilter membrane is made.

11. The method of any one of claims 9 or 10, wherein the drying is performed until the intermediate solvent is below a limit of detection by ATR-FTIR spectroscopy.

12. The method of any one of the preceding claims, wherein the drying is performed until the amount of storage solution in the matrix is no more than 1% of saturation.

13. The method of any one of the preceding claims, wherein the test fluid comprises or consists of an organic solvent or mixture of organic solvents.

14. The method of any one of the preceding claims, wherein the microfilter membrane comprises hollow fibers, and wherein the applying the pressure comprises limiting the pressure to a level that does not cause bursting of the fibers of the microfilter membrane.

15. The method of any one of the preceding claims, wherein the microfilter membrane comprises a flat sheet membrane, and wherein the applying the pressure comprises limiting the pressure to a level that does not cause bursting or rupture of the flat sheet membrane of the microfilter membrane.

## Patentansprüche

1. Ein Verfahren zur Bestimmung eines Porengrößenprofils eines Mikrofilters, wobei das Verfahren umfasst:
Bereitstellen (210) einer gesättigten porösen Mikrofiltermembran, die eine erste Oberfläche, eine zweite Oberfläche und eine dazwischen angeordnete Matrix umfasst, wobei die Matrix mit einer Lagerungslösung gesättigt ist, wobei die Lagerungslösung eine wasserlösliche, nichtflüchtige Lösung umfasst oder aus einer solchen besteht, bei der es sich um ein Polyol, vorzugsweise Glycerin, handelt;
Inkontaktbringen (220) der gesättigten porösen Mikrofiltermembran durch Eintauchen in ein Zwischenlösungsmittel, wodurch sich die Lagerungslösung in dem Zwischenlösungsmittel löst und die Membran dadurch entsättigt wird;
Aufbringen (230) einer Testflüssigkeit auf die entsättigte Membran, wodurch die Membran wieder gesättigt wird;
Ausüben (240) eines Drucks auf die erste Oberfläche der wieder gesättigten Membran, wobei der Druck durch Inkontaktbringen der ersten Oberfläche mit einem Gas oder einer Flüssigkeit ausgeübt wird;
Erfassen (250) eines Flusses des Gases, der Flüssigkeit und/oder der Testflüssigkeit von der zweiten Oberfläche als Reaktion auf den genannten Druck; und
Bestimmen (260) des Porengrößenprofils der porösen Mikrofiltermembran auf der Grundlage eines Druckniveaus, das zu dem Durchfluss des Gases, der Flüssigkeit und/oder der Testflüssigkeit von der zweiten Oberfläche führt,
wobei das Entsättigen der Mikrofiltermembran darin besteht, dass die Lagerungslösung unterhalb einer Nachweisgrenze mittels ATR-FTIR-Spektroskopie liegt.

2. Ein Verfahren zum Filtern eines Zellkulturprodukts, das ein therapeutisches Protein umfasst, wobei das Verfahren das Verfahren zum Bestimmen eines Porengrößenprofils eines Mikrofilters gemäß Anspruch 1 umfasst und ferner:
Auswählen (271) der Membran zur Verwendung beim Filtern nur dann, wenn das ermittelte Porengrößenprofil der Membran innerhalb eines festgelegten Bereichs liegt, wobei der festgelegte Bereich den Durchgang des therapeutischen Proteins durch die poröse Mikrofiltermembran ermöglicht; und
Filtern (281) des das therapeutische Protein enthaltenden Zellkulturprodukts durch die ausgewählte Mikrofiltermembran oder eine Mikrofiltermembran aus derselben Charge wie die ausgewählte Mikrofiltermembran.

3. Das Verfahren nach Anspruch 2, wobei der festgelegte Bereich den Durchgang von Molekülen mit einem Molekulargewicht von bis zu 750 kDa durch die poröse Mikrofiltermembran ermöglicht.

4. Das Verfahren nach Anspruch 2 oder 3, wobei der festgelegte Bereich 5 - 120 Nanometer beträgt.

5. Das Verfahren nach einem der Ansprüche 2 bis 4, wobei das therapeutische Protein aus der Gruppe ausgewählt ist, bestehend aus: einem Antikörper, einem Antigen-bindenden Antikörperfragment, einem Antikörperproteinprodukt, einem bispezifischen T-Zell-Engager (BiTE^{®})-Molekül, einem multispezifischen Antikörper, einem Fc-Fusionsprotein, einem rekombinanten Protein und einem aktiven Fragment eines rekombinanten Proteins.

6. Das Verfahren nach einem der vorstehenden Ansprüche, wobei die Mikrofiltermembran Polysulfon, Polyethersulfon, Polyvinylidenfluorid oder Cellulose umfasst.

7. Das Verfahren nach Anspruch 1 oder 2, wobei das Polyol Glycerol, wie beispielsweise Glycerin, umfasst.

8. Das Verfahren nach einem der vorstehenden Ansprüche, wobei das Zwischenlösungsmittel einen Alkohol, wie beispielsweise Isopropylalkohol, umfasst.

9. Das Verfahren nach einem der vorstehenden Ansprüche, das ferner das Trocknen der entsättigten Mikrofiltermembran vor dem Aufbringen der Testflüssigkeit umfasst.

10. Das Verfahren nach einem der vorstehenden Ansprüche, wobei das Entsättigen der Mikrofiltermembran darin besteht, dass die Lagerungslösung unterhalb einer Nachweisgrenze mittels ATR-FTIR-Spektroskopie liegt, wobei ein ATR-FTIR-Spektrum der ersten und/oder zweiten Oberfläche mit einem Referenzspektrum des reinen Materials übereinstimmt, aus dem die Mikrofiltermembran hergestellt ist.

11. Das Verfahren nach einem der Ansprüche 9 oder 10, wobei das Trocknen so lange durchgeführt wird, bis das Zwischenlösungsmittel unterhalb einer Nachweisgrenze mittels ATR-FTIR-Spektroskopie liegt.

12. Das Verfahren nach einem der vorstehenden Ansprüche, wobei das Trocknen so lange durchgeführt wird, bis die Menge an Lagerungslösung in der Matrix nicht mehr als 1 % der Sättigung beträgt.

13. Das Verfahren nach einem der vorstehenden Ansprüche, wobei die Testflüssigkeit ein organisches Lösungsmittel oder ein Gemisch aus organischen Lösungsmitteln umfasst oder daraus besteht.

14. Das Verfahren nach einem der vorstehenden Ansprüche, wobei die Mikrofiltermembran Hohlfasern umfasst und wobei das Ausüben des Drucks das Begrenzen des Drucks auf ein Niveau umfasst, das kein Bersten der Fasern der Mikrofiltermembran verursacht.

15. Das Verfahren nach einem der vorstehenden Ansprüche, wobei die Mikrofiltermembran eine Flachmembran umfasst und wobei das Ausüben des Drucks das Begrenzen des Drucks auf ein Niveau umfasst, das kein Bersten oder Reißen der Flachmembran der Mikrofiltermembran verursacht.

## Revendications

1. Procédé de détermination du profil de taille des pores d'un microfiltre, le procédé comprenant :
la fourniture (210) d'une membrane de microfiltre poreuse saturée comprenant une première surface, une deuxième surface et une matrice disposée entre elles, dans lequel la matrice est saturée par une solution de stockage, dans lequel la solution de stockage consiste en, ou comprend, une solution non volatile soluble dans l'eau, qui est un polyol, de préférence de la glycérine ;
la mise en contact (220) de la membrane de microfiltre poreuse saturée par immersion dans un solvant intermédiaire, moyennant quoi la solution de stockage se dissout dans le solvant intermédiaire, désaturant ainsi la membrane ;
l'application (230) d'un fluide d'essai à la membrane désaturée, resaturant ainsi la membrane ;
l'application (240) d'une pression à la première surface de la membrane resaturée, dans lequel la pression est appliquée par la mise en contact de la première surface avec un gaz ou un liquide ;
la détection (250) d'un écoulement de gaz, de liquide et/ou de fluide d'essai provenant de la deuxième surface en réponse à ladite pression ; et
la détermination (260) du profil de taille des pores de la membrane de microfiltre poreuse sur la base d'un niveau de pression qui entraîne l'écoulement du gaz, du liquide et/ou du fluide d'essai provenant de la deuxième surface,
dans lequel la désaturation de la membrane de microfiltre comprend un niveau de solution de stockage en dessous d'une limite de détection par spectroscopie infrarouge à transformée de Fourier à réflectance totale atténuée, ATR-FTIR.

2. Procédé de filtration d'un produit de culture de cellules comprenant une protéine thérapeutique, le procédé comprenant le procédé de détermination d'un profil de taille des pores d'un microfiltre selon la revendication 1, et en outre :
la sélection (271) de la membrane à utiliser pour la filtration uniquement si le profil de taille des pores déterminé de la membrane se situe dans une plage spécifiée, dans lequel la plage spécifiée permet le passage de la protéine thérapeutique à travers la membrane de microfiltre poreuse ; et
la filtration (281) du produit de culture de cellules comprenant la protéine thérapeutique à travers la membrane de microfiltre sélectionnée ou une membrane de microfiltre du même lot que la membrane de microfiltre sélectionnée.

3. Procédé selon la revendication 2, dans lequel la plage spécifiée permet le passage de molécules ayant une masse moléculaire allant jusqu'à 750 kDa à travers la membrane de microfiltre poreuse.

4. Procédé selon la revendication 2 ou 3, dans lequel la plage spécifiée est de 5 à 120 nanomètres.

5. Procédé selon l'une quelconque des revendications 2 à 4, dans lequel la protéine thérapeutique est sélectionnée dans le groupe constitué par : un anticorps, un fragment d'anticorps se liant à un antigène, un produit protéique d'anticorps, une molécule d'anticorps bispécifique d'engagement des lymphocytes T (BiTE^{®}), un anticorps multispecifique, une protéine de fusion Fc, une protéine recombinante, et un fragment actif d'une protéine recombinante.

6. Procédé selon l'une quelconque des revendications précédentes, dans lequel la membrane de microfiltre comprend un polysulfone, un polyéthersulfone, du polyfluorure de vinylidène ou de la cellulose.

7. Procédé selon la revendication 1 ou 2, dans lequel le polyol comprend du glycérol, soit de la glycérine.

8. Procédé selon l'une quelconque des revendications précédentes, dans lequel le solvant intermédiaire comprend un alcool, tel que l'alcool isopropylique.

9. Procédé selon l'une quelconque des revendications précédentes, comprenant en outre le séchage de la membrane de microfiltre désaturée avant l'application du fluide d'essai.

10. Procédé selon l'une quelconque des revendications précédentes, dans lequel la désaturation de la membrane de microfiltre comprend un niveau de solution de stockage en dessous d'une limite de détection par spectroscopie ATR-FTIR, dans lequel un spectre ATR-FTIR de la première surface et/ou de la deuxième surface correspond à un spectre de référence du matériau pur dont est constituée la membrane de microfiltre.

11. Procédé selon l'une quelconque des revendications 9 et 10, dans lequel le séchage est effectué jusqu'à ce que le solvant intermédiaire soit inférieur à la limite de détection par spectroscopie ATR-FTIR.

12. Procédé selon l'une quelconque des revendications précédentes, dans lequel le séchage est effectué jusqu'à ce que la quantité de solution de stockage dans la matrice ne dépasse pas 1 % de la saturation.

13. Procédé selon l'une quelconque des revendications précédentes, dans lequel le fluide d'essai consiste en, ou comprend, un solvant organique ou un mélange de solvants organiques.

14. Procédé selon l'une quelconque des revendications précédentes, dans lequel la membrane de microfiltre comprend des fibres creuses, et dans lequel l'application de la pression comprend la limitation de la pression à un niveau qui ne provoque pas l'éclatement des fibres de la membrane de microfiltre.

15. Procédé selon l'une quelconque des revendications précédentes, dans lequel la membrane de microfiltre comprend une membrane à feuille plane, et dans lequel l'application de la pression comprend la limitation de la pression à un niveau qui ne provoque pas l'éclatement ou la rupture de la membrane à feuille plane de la membrane de microfiltre.
